# FASCICULE DE BREVET EUROPEEN

(11) **EP 0 357 656 B1**
(45) Date de publication et mention de la délivrance du brevet: **29.12.1993**
(21) Numéro de dépôt: 88903871.7
(22) Date de dépôt: 02.05.1988
(51) Int. Cl.: A61B 17/42, A61B 17/425, A61D 19/02

(54) **PROCEDE DE FECONDATION INTRA-UTERINE DE MAMMIFERE ET DISPOSITIF POUR SA MISE EN OEUVRE**
VERFAHREN UND VORRICHTUNG ZUR INTRAUTERINEN BEFRUCHTUNG
PROCESS FOR INTRA-UTERINE FERTILIZATION IN MAMMALS AND DEVICE FOR IMPLEMENTATION THEREOF

(30) Priorité: 30.04.1987 FR 8706205
(43) Date de publication de la demande: 14.03.1990
(73) Titulaire: RANOUX, Claude, Lynn, MA 01904 (US)
(72) Inventeur: RANOUX, Claude, Lynn, MA 01904 (US)
(74) Mandataire: CABINET BONNET-THIRION
(86) Numéro de dépôt international: FR8800212
(87) Numéro de publication internationale: WO8808280

(56) Documents cités:
- EP-A- 0 066 488
- WO-A-83/02386
- DE-A- 1 616 899
- FR-A- 2 539 628
- FR-A- 2 589 879
- US-A- 3 910 275
- US-A- 4 380 997
- The Lancet. Saturday 8 May 1982; page 1031-1033; "Human pregnancy following oocyte and sperm transfer to the uterus", Ian Craft et al

## Description

La présente invention concerne un procédé de fécondation de mammifère et un dispositif pour sa mise en oeuvre.

Le demandeur à déjà décrit un procédé de fécondation humaine faisant appel à un conteneur dans les demandes de brevet FR-A-2589879 et PCT/FR 86 00378.

Dans ce procédé le prélèvement des ovocytes d'une patiente est effectué d'une manière classique le plus souvent sous contrôle échographique. La patiente subit au préalable une stimulation ovarienne par du Clomid-HMG ou par des agonistes de la LH-RH tels la busèréline et le DTR-P-6.

Les liquides folliculaires sont aspirés à la seringue et immédiatement examinés au Laboratoire.

On place ensuite jusqu'à 8 de ces ovocytes dans un conteneur constitué par un tube ouvert à l'une de ses extrémités et rempli jusqu'à ras bord d'un milieu de culture, par exemple milieu B2 de MENEZZO, contenant 10 000 à 20 000 spermatozoïdes humains mobiles par millilitre. Puis on ferme hermétiquement le tube, sans interposition d'air, c'est-à-dire sans couche d'air au-dessus de la phase liquide.

Le tube fermé est ensuite replacé dans la cavité vaginale de la patiente pendant 44 à 50 heures. Le tube est ensuite récupéré, ouvert et les ovocytes sont examinés au Laboratoire.

Les ovocytes fécondés, ayant subi les premiers clivages sont des embryons dans leurs premiers stades de développement.

Ces embryons jusqu'au nombre de 4, sont alors replacés dans la cavité utérine de la patiente, à l'aide d'un cathéter de Frydman.

Cette technique dénommée CIVETE (Culture intra vaginale et transplantation embryonnaire) a permis de constater que les taux de clivage, de transfert et de naissance par ponction étaient au moin égaux à ceux obtenus en FIVETE (Fécondation in vitro et transplantation embryonnaire).

Dans la CIVETE jusqu'à 8 ovocytes sont placés dans un conteneur contenant un volume de 3,2 ml de liquide constitué par du milieu B2 contenant de 10 000 à 20 000 spermatozoîdes mobiles par millilitre. Ainsi le volume moyen de liquide par ovocyte est de 400 microlitres contenant de 4 000 à 8 000 spermatozoïdes mobiles.

La CIVETE bien que présentant de nombreux avantages par rapport à la FIVETE, car ne nécessitant pas de couveuse avec une atmosphère d'air enrichi de CO₂, présente cependant encore des inconvénients. Les étapes de manipulation des gamètes mâles et femelles sont encore nombreuses et la technique nécessite en particulier un replacement du ou des embryons dans l'utérus. Ces nombreuses manipulations sont des causes de toxicité ou même de perte d'embryons.

Ian Craft et al ont décrit dans un article intitulé "Human Pregnancy Following oocyte and Sperm Transfer to the Uterus" dans la revue The Lancet du 8 mai 1982 un procédé de fécondation dans lequel l'ovocyte entouré de son cumulus est incubé à 37°C dans un milieu de culture pendant 6 heures avant d'ajouter des spermatozoïdes. On continue l'incubation à 37°C pendant une heure. Ensuite on débarrasse l'ovocyte de son cumulus et on le transfère avec du milieu de culture contenant des spermatozoïdes dans un cathéter. L'extrémité du cathéter est introduite dans l'utérus et son contenu y est injecté. Ce procédé fait appel à deux conteneurs différents successifs: une coupelle dans laquelle l'ovocyte est incubé pendant 7 heures et un cathéter pour prélever l'ovocyte, spermatozoïdes et milieu de culture et les transférer dans l'utérus. Dans ce procédé on utilise le cathéter pour effectuer le transfert de l'ovocyte et des spermatozoïdes dans l'utérus avant que la fécondation et le clivage de l'ovocyte en embryon n'aient eu lieu.

Afin de remédier aux inconvénients sommairement évoqués ci-dessus le Demandeur propose un procédé de fécondation de mammifère caractérisé en ce qu'il comporte les étapes suivantes:
a) on prévoit un conteneur apte à permettre son introduction et son logement dans la cavité utérine du mammifère ;
b) on remplit ce conteneur avec un milieu de culture, au moins un ovocyte du mammifère, et des spermatozoïdes ;
c) on insère ce conteneur rempli dans la cavité utérine;
d) on laisse le conteneur dans la cavité utérine pendant une durée déterminée pour obtenir la fécondation du ou des ovocytes dans le conteneur ;
e) on permet l'échappement du contenu du conteneur dans la cavité utérine.

Ce procédé évite en particulier l'étape finale de transfert embryonnaire de l'extérieur du mammifère à l'intérieur de sa cavité utérine.

Selon un aspect préféré du procédé suivant l'invention on s'arrange pour que l'échappement soit fait par éjection du contenu du conteneur dans le fond de la cavité utérine de façon à augmenter les chances de nidation des ovocytes fécondes ou embryons.

De même dans le cas d'un conteneur en matériau biodégradable on préfère l'insérer vers le fond de l'utérus pour les mêmes raisons que ci-dessus.

Cependant ce procédé nécessite un conteneur d'une taille appropriée à son introduction et son logement dans la cavité utérine du mammifère. Cette taille doit être notablement plus faible que celle d'un conteneur destiné à la FIVETE car, en particulier chez la femme, le col de l'utérus ne peut présenter qu'une ouverture de l'ordre de quelques millimètres.

Pour arriver au procédé proposé ci-dessus le Demandeur a entrepris dès décembre 1986 par des études préalables, de mettre en oeuvre un procédé comportant les étapes suivantes :
1° on prévoit une paillette fine d'une contenance d'environ 250 microlitres ;
2° on remplit cette paillette avec un milieu de culture, au moins un ovocyte humain et des spermatozoïdes mobiles humains;
3° on bouche cette paillette à au moins une de ses extrémités l'autre baignant dans un récipient contenant du milieu de culture ;
4° on place cette paillette remplie et son récipient dans une étuve à 37° pendant 20 à 30 heures ;
5° on recueille les embryons qui sont replacés en culture dans du milieu de culture à l'étuve à 37° jusqu'au lendemain ;
6° on replace un ou plusieurs embryons dans la cavité utérine de la patiente à l'aide d'un cathéter de Frydman.

Le(s) embryon(s) sont replacés 44 à 50 heures après avoir rempli la paillette du mélange milieu de culture, ovocyte(s), spermatozoïdes.

Ces études préalables ont montré que dans des essais portant sur 57 ovocytes, 33 étaient fécondés pour donner des embryons à replacer, (soit un taux de clivage d'environ 58%), tandis que 88 ovocytes prélevés d'une manière concomittante aux prélèvements des 57 ovocytes conduisaient par la fécondation in vitro (FIV) ou par la culture intra vaginale (CIV) à 44 embryons à replacer soit un taux de clivage de 50%. Deux grossesses peuvent être attribuées avec certitude au replacement d'ovocytes fécondés en paillette fine.

Ces études préalables ont montré que l'on pouvait féconder dans une telle paillette jusqu'à 4 ovocytes humains. Il a été ainsi trouvé que cette fécondation en paillettes fines nécessitait seulement un volume moyen de milieu de culture par ovocyte d'environ 60 microlitres contenant de 600 à 1200 spermatozoïdes mobiles. Le milieu de culture choisi dans ces études a été du B2 de Menezzo.

La présente invention prévoit pour la mise en oeuvre du procédé de fécondation, un dispositif de fécondation de mammifère dans lequel le conteneur comprend un tube fermé à une de ses extrémités dite inférieure, caractérisé en ce que ledit tube est solidarisé vers son extrémité inférieure à un dispositif de maintien apte à être fixé d'une manière amovible au col de l'utérus du mammifère, après passage du tube par l'ouverture du col de l'utérus et logement du tube dans l'utérus.

Le dispositif de maintien évite que le tube ne se perde dans la cavité utérine et de plus permet de bien positionner la zone de sortie du tube vers le fond de l'utérus pour augmenter les chances d'une nidation.

Selon une autre caractéristique préférée de l'invention, ce tube est solidarisé au dispositif de maintien à la faveur d'un alésage longitudinal traversant celui-ci.

Ces caractéristiques permettent d'éjecter le contenu du conteneur au moment choisi par l'opérateur en agissant comme cela sera décrit par la suite sur un bouchon formant piston à l'extrémité inférieure. Comme le dispositif de maintien dépasse en pratique de l'utérus, ce bouchon est facilement accessible par la voie naturelle.

La présente invention prévoit également pour la mise en oeuvre du procédé de fécondation un dispositif de fécondation de mammifère dans lequel le conteneur comprend un tube, fermé à l'une au moins de ses extrémités, caractérisé en ce que le tube comporte une paroi en un matériau biodégradable en un temps déterminé par son contenu prévu, à savoir ovocyte, spermatozoïdes et milieu de culture et par un environnement prévu intra-utérin, le tube ayant une longueur inférieure ou égale à la profondeur de la cavité utérine du mammifère.

Selon une caractéristique préférée de l'invention le tube est entièrement en un matériau biodégradable. Cela a l'avantage de pouvoir introduire dans la cavité utérine le conteneur rempli de gamètes et de milieu de culture sans intervention supplémentaire pour obtenir une nidation du ou des embryons.

La présente invention sera mieux comprise à l'aide de la description qui va suivre en référence aux dessins annexés où:
les figures 1 a, b, c, d, e, f, montrent une vue schématique agrandie d'une première forme de réalisation d'un conteneur selon l'invention formé à partir d'un tube d'un matériau biodégradable et l'insertion de ce conteneur rempli dans la cavité utérine d'une patiente.
les figures 2a, b, c, montrent une seconde forme de réalisation d'un conteneur selon l'invention formé à partir d'une feuille mince d'un matériau biodégradable ;
la figure 3 montre d'une manière schématique une vue en coupe longitudinale d'une troisième forme de réalisation d'un conteneur associé à un dispositif de maintien au col de l'utérus du mammifère ;
la figure 4 montre une vue en coupe longitudinale d'une variante de réalisation du conteneur associé au dispositif de maintien de la figure 3, avec un bouchon biodégradable ;
la figure 5 montre une vue en coupe longitudinale d'une autre forme de réalisation d'un conteneur associé à un dispositif de maintien au col de l'utérus du mammifère;
la figure 6 montre une variante de réalisation du conteneur et de son dispositif de maintien associé de la figure 5 .
la figure 7 montre d'une manière schématique le conteneur et son dispositif de maintien de la figure 3, le conteneur étant logé dans l'utérus et maintenu en place par le dispositif de maintien fixé au col de l'utérus.
la figure 8 montre d'une manière schématique l'étape du procédé suivant l'invention ou le conteneur de la figure 3 est vidé de son contenu dans la cavité utérine.

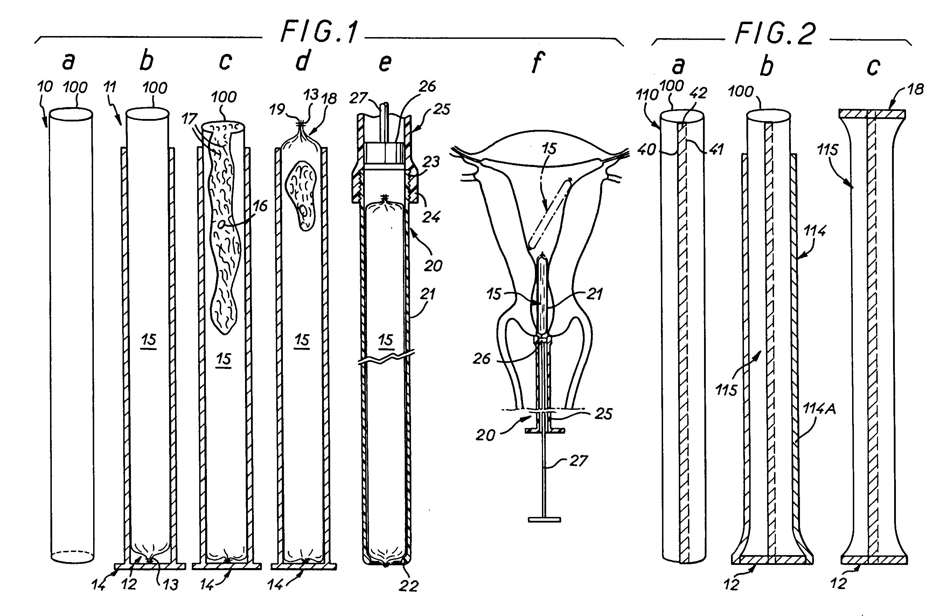

La figure 1a montre, agrandi, un tube cylindrique 10 d'une longueur plus petite que la profondeur utérine. Ce tube a été obtenu par extrusion et il possède une paroi 100 d'épaisseur constante.

Le matériau qui le constitue est un polymère du règne animal ou végétal, en particulier un polymère hydrophile par exemple du collagène, du fibrinogène ou un sucre polymère.

La figure 1b montre un tube 11 fermé à son extrémité inférieure 12 par une ligature d'un fil 13 et obtenu à partir du tube ouvert 10. Ce fil 13 est de préférence biodégradable et même résorbable par l'organisme d'un mammifère. De tels fils sont employés en chirurgie comme suture résorbable. La ligature confère la qualité de conteneur 15 au tube 11, son extrémité inférieure 12 ayant une forme en extrémité de saucisse la fermeture du tube 10 à ses extrémités inférieure et supérieure peut être aussi réalisée par soudure, ou par collage à l'aide d'une colle biodégradable ou encore par simple pression. La zone de sortie est définie ici par la paroi 100 en un matériau biodégradable, en un temps déterminé, par le contenu prévu (milieu de culture, spermatozoïdes, ovocyte(s) et son environnement prévu intra-utérin. Le conteneur 15 est ici entièrement en un matériau biodégradable en un temps déterminé.

Le tube 11 a été glissé à l'intérieur d'une éprouvette à pied 14 ayant un diamètre intérieure sensiblement égal au diamètre extérieur du tube 11 et qui lui sert de support.

Cette éprouvette à pied peut être en un matériau connu pour ses propriétés de faible frottement comme le téflon ou du polyéthylène, et est d'une hauteur légèrement inférieure à la longueur du tube fermé 11.

la figure 1c montre avec arrachement le conteneur 15 rempli à ras bord par un milieu de culture, ici du milieu B2 de Ménézzo, et contenant un ovocyte 16, et des spermatozoïdes 17. L'éprouvette 14 permet de contrebalancer la pression hydrostatique du liquide remplissant le conteneur 15 et ainsi d'éviter une rupture prématurée de la paroi si mince soit t'elle. Ce conteneur rempli et fermé seulement à une de ses extrémités 12 peut également être laissé ainsi l'autre extrémité restant ouverte pour une insertion dans la cavité utérine dans la mesure ou son contenu ne se vide pas spontanément lorsque le tube est retourné son extrémité ouverte dirigée vers le bas.

Le remplissage du conteneur peut se faire d'une manière classique à l'aide d'une seringue dont le diamètre intérieur de l'aiguille est supérieur à celui de l'ovocyte.

Le remplissage du tube 10 pour aboutir au tube rempli de la figure 1c peut se faire également par aspiration de la manière suivante :

Le tube 10 ouvert à ses deux extrémités est engagé à l'intérieur d'un cylindre support creux allongé ayant un diamètre intérieur égal à celui de l'éprouvette 14 de la figure 1b, mais ouvert à ses deux extrémités de façon à ce que le tube 10 dépasse en longueur les deux extrémités du cylindre creux. Un embout d'aspiration est alors fixé sur le haut du cylindre d'une manière étanche de façon à ne pas provoquer un pliage de l'extrémité supérieure du tube 10. L'extrémité inférieure du tube 10 et celle du cylindre support sont alors plongées dans un milieu de culture B2 contenant des spermatozoïdes et un ou plusieurs ovocytes. On aspire ces gamètes et le milieu de culture de manière à remplir le tube 10 ouvert à ses deux extrémités. On sort les extrémités inférieures du liquide et on ferme celle inférieure du tube 10 par ligature, collage ou soudage avant d'enlever l'embout d'aspiration du haut du cylindre support. On obtient ainsi un tube rempli, analogue à celui représenté à la figure 1c sauf que l'éprouvette 14 a été remplacée par un cylindre creux ouvert à ses deux extrémités.

La figure 1d montre une vue avec arrachement du conteneur 15 de la figure précédente fermé vers son extrémité supérieure 18 par une ligature, à l'aide d'un même fil résorbable 13. Cette façon d'opérer permet d'enfermer dans le conteneur 15 fermé à ses deux extrémités 12, 18 un liquide sans interposition d'air, si cela est désiré.

En effet pendant le déroulement de la ligature 18, une partie du liquide excédentaire reste dans la partie tronconique 19 du tube 10 situé au dessus de la ligature supérieure.

L'épaisseur de la paroi 100 du tube 10 en matériau biodégradable joue un rôle essentiel. En effet cette paroi dans le procédé suivant l'invention doit être apte à se désagréger au bout d'un certain temps après introduction du conteneur saucisse 15 de la figure 1d à l'intérieur de la cavité utérine du mammifère, afin de laisser s'échapper spontanément son contenu dans la cavité utérine après une durée déterminée et donc de pouvoir permettre la nidation du ou des embryons. Cet échappement peut être facilité par la présence d'une extrémité du tube 11 restée ouverte dans le cas où une seule des extrémités du tube 10 a été fermée.

Le matériau du conteneur doit être résorbable et non immunogène. Ici le conteneur a une paroi d'épaisseur unique, mais il est également possible de prévoir un conteneur biodégradable à parois d'épaisseur différente.

L'utérus d'un mammifère procure à la cavité utérine une ambiance chaude et humide, mais sans grande activité enzymatique susceptible de provoquer la dégradation du matériau biodégradable.

Par contre les spermatozoïdes mobiles de mammifère possèdent une très forte activité enzymatique susceptible de produire une attaque interne de la paroi 100 du conteneur 15 allant jusqu'à sa dégradation et donc son ouverture.

Pour déterminer l'épaisseur de la paroi adéquate pour avoir un échappement du contenu après la durée déterminée qui par exemple pourra être de 15 à 55 heures on réalise l'essai suivant in vitro:
On produit un conteneur ayant une épaisseur de paroi égale à E et rempli selon la figure 1d d'un milieu de culture contenant de 10 000 à 20 000 spermatozoïdes par millilitre, mais sans ovocyte.

Ce conteneur ainsi rempli est disposé dans une étuve saturée de vapeur d'eau à une température sensiblement égale à celle interne du mammifère. On observe régulièrement l'état du conteneur et on note le moment où le contenu s'échappe spontanément du conteneur. Si le temps nécessaire pour observer cet échappement est supérieure à 55 heures on produit un autre conteneur dont la paroi à une épaisseur inférieure à E soit par exemple 0,5 E et on soumet ce conteneur au test in vitro ci-dessus et ainsi de suite par itération jusqu'à obtenir une paroi 100 ayant l'épaisseur déterminée conduisant à la durée déterminée voulue, par exemple de 15 à 55 heures in vitro.

L'épaisseur de la paroi 100 du tube 10, 11 en matériau biodégradable peut être comprise entre 0,01 et 1 mm.

Des essais ont montré qu'un milieu de culture B2 de Menezzo contenant de 10 000 à 20 000 spermatozoïdes par millilitre dégradait dans cet essai in vitro en plus de 72 heures une paroi de collagène tané d'une épaisseur de 0,1 mm et en 15 minutes une paroi de gélose d'une épaisseur de 0,1 mm servant de matériau constitutif d'une capsule gastro-résistante.

La figure 1e montre le conteneur 15 rempli selon la figure 1d et qui a été séparé de son éprouvette 14 de soutien avant d'être introduit dans un dispositif d'implantation 20 comprenant un tuyau cylindrique 21 dont le diamètre intérieur est sensiblement égal à celui de l'éprouvette 14. La longueur du tuyau cylindrique 21 est supérieure à celle du conteneur 15 et peut être par exemple voisine ou supérieure à la profondeur de la cavité utérine du mammifère.

Ce tuyau cylindrique 21 comporte à son extrémité inférieure un bord arrondi 22 qui réduit légèrement le diamètre de son orifice afin d'éviter le glissement spontané du conteneur 15 en le retenant légèrement. Le tuyau 21 comporte vers son extrémité supérieure un filetage 23 extérieur au tuyau sur lequel vient se visser l'embout 24 d'un tuyau cylindrique 25 muni à l'intérieur d'un piston 26 coulissant à frottement doux et solidarisé à la tige 27. Le diamètre intérieur de ce cylindre 25 est égal à celui du diamètre intérieur du tuyau 21 afin de permettre la continuité du coulissement du piston 26 dans le tuyau cylindrique 21.

La figure 1f montre une vue schématique de l'introduction et le logement du conteneur 15 dans la cavité utérine d'un mammifère par pénétration du tuyau 21 dans la cavité utérine en franchissant le col de l'utérus.

Le tuyau 21 est introduit dans la cavité utérine de façon à laisser suffisamment de profondeur disponible pour le, logement du conteneur rempli 15, puis le piston 26 est poussé grâce à la tige 27 qui dépasse du corps du mammifère comme d'ailleurs le tuyau cylindrique 25, pour déposer à l'intérieur de la cavité utérine vers le fond de l'utérus le conteneur 15. Ensuite le dispositif d'implantation 20 est retiré de l'utérus et du vagin du mammifère et l'échappement du contenu du conteneur 15 rempli s'effectue après un délai déterminé par la biodégradation d'au moins une partie du conteneur.

Les figures 2 a, b, c montrent la réalisation d'un autre conteneur en un matériau biodégradable.

La figure 2a montre la constitution d'un tube creux 110 allongé à partir d'une bande de faible épaisseur d'un matériau biodégradable. Cette bande peut par exemple être roulée sur un cylindre plein de mise en forme (non représenté) et les bords 40 et 41 peuvent se dépasser légèrement pour assurer une zone de contact 42 commune de collage à l'aide d'une colle biodégradable ou de soudage par serrage ou de soudage à chaud.

Ce tube possède une paroi 100 de faible épaisseur, la zone commune 42 étant plus épaisse.

Ce tube 110 peut, comme le tube 10 précédent, être rempli par aspiration d'un milieu de culture contenant des spermatozoïdes et au moins un ovocyte.

La figure 2b montre que l'extrémité inférieure du tube 110 précédent a été fermée par serrage ou collage pour constituer un conteneur 115 qui peut être disposé dans un support 114 évasé vers le bas et dépourvu de fond.

La hauteur du support 114 est légèrement inférieure à la longueur du conteneur 115.

Le support est par ailleurs séparable en deux demi-coques 114A de façon à pouvoir libérer le conteneur 115 qu'il soutient.

La figure 2c montre le conteneur 115 rempli, fermé également par serrage ou collage à son extrémité supérieure 18 et apte à être inséré dans la cavité utérine du mammifère à l'aide du dispositif d'implantation analogue au dispositif d'implantation 20.

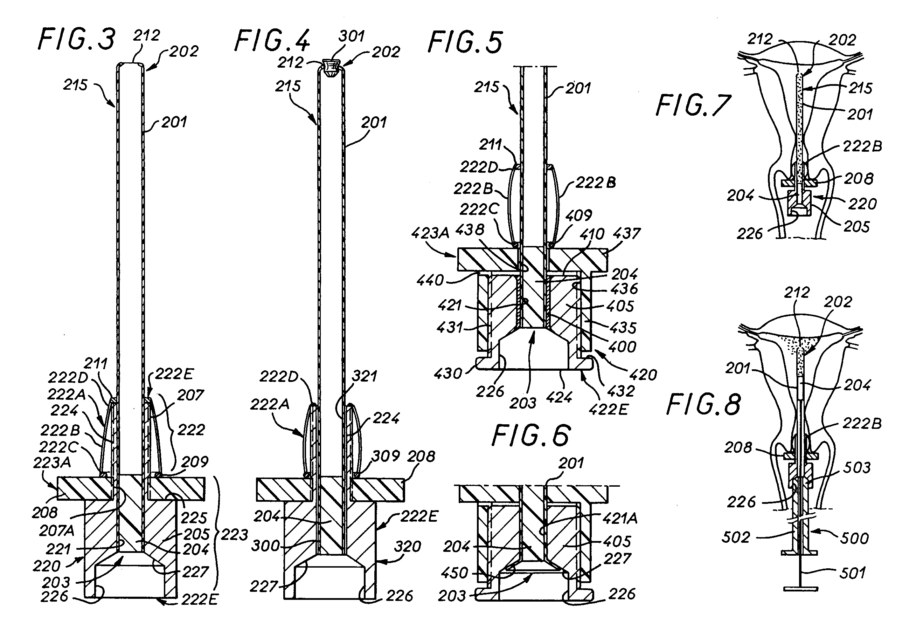

La figure 3 montre un conteneur 215 associé à un dispositif de maintien 220 apte à être fixé d'une manière amovible au col de l'utérus du mammifère. Ce conteneur est constitué par une paillette ou tube 201 réalisé dans un matériau synthétique non biodégradable par les spermatozoïdes, par exemple en polyéthylène ou polypropylène non toxique pour les spermatozoïdes et les ovocytes ni d'ailleurs pour le mammifère.

Le tube est souple et non cassant pour pouvoir se déformer lors du passage difficile du col de l'utérus et s'adapter à la forme interne de la cavité utérine du mammifère. Ce tube 201 a une extrémité dite supérieure 202 ouverte pouvant être éventuellement fermée par un petit bouchon (non représenté) en matière biodégradable par exemple collagène, fibrinogène, sucre polymère.

L'extrémité ouverte 202 présente un orifice rétréci 212 par rapport au diamètre intérieur du tube 201.

L'autre extrémité dite inférieure 203 du tube est fermée par un bouchon 204 apte à former un piston étanche à l'intérieur de la paillette 201.

La longueur du tube 201 est du même ordre que la profondeur de la cavité utérine du mammifère.

Pour une patiente la longueur du tube 201 peut être comprise entre 5 et 7 cm et le diamètre extérieur jusqu'à 3 mm, afin de pouvoir glisser le tube sans traumatisme dans la cavité utérine par le col de l'utérus.

Le tube 201 est solidarisé sur une partie de sa longueur, vers son extrémité inférieure 203, au dispositif de maintien 220 à la faveur d'un alésage longitudinal traversant 221 de celui-ci.

Le dispositif de maintien 220 comporte successivement dans le sens longitudinal une première partie 222, apte à être introduite dans le col de l'utérus et une seconde partie 223 apte à être disposée dans le vagin à l'extérieur du col et en butée contre celui-ci, la première partie comportant un élément 222A extensible radialement ayant une position de repos pour l'insertion dans le col et une deuxième position dans laquelle il s'appuie sur les parois internes du col de l'utérus.

L'élément extensible 222A est formé d'un matériau flexible a paroi mince et apte à s'étendre radialement dans la deuxième position.

L'élément extensible comporte au moins deux languettes 222B sensiblement longitudinales, chaque languette 222B ayant une extrémité inférieure 222C mobile et une extrémité supérieure 222D fixe longitudinalement par rapport au tube 201.

Le dispositif de maintien 220 comporte en outre une partie mobile 223A pour contrôler la position longitudinale de l'extrémité inférieure 222C de l'élément extensible 222A et une partie fixe 222E par rapport au tube 201 sur laquelle la partie mobile 223A est adaptée à se déplacer.

D'une manière détaillée la partie fixe 222E comporte une extension longitudinale cylindrique 224 appartenant à ladite première partie 222 du dispositif de maintien 220, extension 224 qui comporte sur sa surface externe un pas de vis 207.

L'extension cylindrique 224 est d'un seul tenant avec un manchon cylindrique 205 de plus grand diamètre et leur réunion forme un épaulement 225 appartenant à la face extérieure supérieure du manchon 205.

L'extension 224 et le manchon 205 sont traversés par un premier alésage 221 d'un diamètre légèrement inférieur à celui extérieur du tube 201 pour que celui-ci puisse y être introduit à force et ensuite maintenu.

Le manchon 205 comporte un second alésage 226 d'un diamètre supérieur à celui extérieur du tube 201, le premier alésage débouchant dans le second par un évidement 227 en tronc de cone de révolution.

Le second alésage 226 à une profondeur et un diamètre adaptés pour recevoir un dispositif poussoir apte à déplacer le bouchon 204 dans le tube 201.

La partie mobile 223A comporte une molette 208 formant écrou grâce à un pas de vis complémentaire 207A au pas de vis 207.

Ainsi d'une manière générale la partie fixe 222E et la partie mobile 223A comportent des pas de vis complémentaires 207, 207A peur permettre à la partie mobile de tourner et de se déplacer longitudinalement par rapport à la partie fixe.

La figure 3 montre que la molette 208 à un diamètre suffisant pour qu'elle puisse être disposée en butée sur le col de l'utérus. La molette en position basse sur le pas de vis complémentaire 207 arrive par une de ses faces en contact avec l'épaulement 225. Sur l'autre face globalement circulaire de la molette repose un anneau 209 dit inférieur entourant l'extension cylindrique 224.

Le tube 201 comporte une bague soudée 211 qui arrive sensiblement en butée sur le haut de l'extension cylindrique 224.

Chaque languette 222B est solidaire par son extrémité inférieure 222C à l'anneau inférieur 209 et par son extrémité supérieure 222D à la bague soudée 211. Ainsi l'extrémité supérieure de l'élément extensible 222A est fixée à une bague 211 soudée au tube 201. Chaque languette possède une très légère courbure définissant une concavité qui est orientée vers l'extension cylindrique 224.

Ainsi tout rapprochement longitudinal de l'anneau 209 et de la bague 211 augmente la courbure des languettes 222B et leur extension radiale, et ainsi la flèche de la courbure.

La figure 4 montre une variante de réalisation du tube 201 associé à un dispositif de maintien 320.

L'extrémité supérieure 222D de l'élément extensible 222A est attachée à l'extrémité supérieure de l'extension cylindrique 224 de la partie fixe 222E ce qui permet de supprimer la bague soudée de la réalisation précédente.

L'alésage longitudinal traversant 321 du dispositif de maintien 320 a un diamètre légèrement supérieur à celui extérieur du tube 201 afin de permettre la solidarisation du tube à la partie fixe 222E par de la colle 300.

L'anneau inférieur 309 qui repose sur la molette 208 est d'une dimension légèrement inférieure à celle de l'anneau 209 de la figure précédente, mais reste toujours apte cependant à coulisser longitudinalement sur l'extension cylindrique 224.

Le tube 201 a l'orifice 212 de sein extrémité supérieure 202 fermée par un bouchon 301 en une matière biodégradable identique à celle décrite ci-dessus. Ce bouchon à une forme adaptée pour être facilement expulsé lors de l'éjection du contenu liquide du conteneur par le déplacement du bouchon 204 formant piston à l'intérieur du tube, lorsque ce bouchon remonte vers l'extrémité 202 supérieure du tube 201.

La figure 5 montre une autre forme de réalisation d'un tube 201 et son dispositif de maintien 420.

Le tube 201 comporte une bague soudée 211 à lui même et rattachée à l'extrémité supérieure 222D de chaque languette 222B dont l'extrémité inférieure 222C est solidaire d'un anneau 409 coulissant en contact avec le tube 201.

En effet dans cette réalisation préférée, l'extension cylindrique des réalisations précédentes a été supprimée.

Ce dispositif de maintien 420 comporte un manchon 405 cylindrique de révolution comportant, partant de sa face circulaire supérieure 410, un alésage axial 421 de profondeur environ égale à la moitié de la hauteur longitudinale du manchon 405.

Cet alésage 421 de longueur l à un diamètre voisin de celui extérieur du tube 201 et permet la solidarisation du manchon 405 au bout inférieur correspondant de longueur l du tube 201 par collage à laide d'une couche de colle 400.

Cet alésage 421 débouche, par un évasement en tronc de cône 227, dans un alésage longitudinal 226 qui débouche sur la face circulaire inférieure 424 du manchon 405. L'alésage 226 et le tronc de cône 227 qui le prolonge sont destinés comme dans les formes de réalisation précédentes aux figures 3 et 4 à recevoir d'une manière amovible des embouts adaptés à transmettre une aspiration si le bouchon 204 qui bouche l'extrémité inférieure 203 du tube 201 est tripartite (bouchon CASSOU des paillettes françaises) ou pour recevoir un embout d'un dispositif poussoir à tige coulissante apte à déplacer le bouchon 204 formant piston à l'intérieur et vers l'extrémité supérieure 202 du tube 201.

Le manchon 405 possède, en prolongement et en alignement avec sa face inférieure 424 une couronne en saillie 430 destinée à faciliter sa préhension.

La face externe cylindrique du manchon 405 est munie sur toute sa hauteur d'un pas de vis 431 qui s'arrête vers le bas à l'épaulement 432 créé par la couronne 430.

Sur le manchon 405 vient se visser une douille 435 dont la surface cylindrique interne comporte un pas de vis 436 complémentaire de celui 431 du manchon 405.

Cette douille 435 a une profondeur longitudinale sensiblement égale à la hauteur du manchon diminuée de la hauteur de la couronne 430.

Le haut de cette douille est fermée par un disque moleté 437 sur sa tranche et d'un seul tenant avec elle-même. Le diamètre du disque est supérieur au diamètre de la douille pour faciliter la préhension du disque moleté. Ce disque 437 est percé d'un passage axial cylindrique 438 dont le diamètre légèrement supérieur à celui extérieur du tube 201 permet un contact glissant avec celui-ci. Pour faciliter un équilibrage de la pression dans la cavité définie entre la face circulaire 410 du manchon 405 et le fond de la douille déterminé par le disque 437, il est prévu un trou 440 traversant la paroi cylindrique de la douille 435 au voisinage immédiat de ce fond.

Ainsi dans cette forme de réalisation la partie fixe 422E et la partie mobile 423A comporte des pas de vis complémentaires 431, 436, peur permettre à la partie mobile de tourner et de se déplacer longitudinalement par rapport à la partie fixe.

Les pas de vis 431, 436 sont respectivement sur une surface externe cylindrique de la partie fixe 422E et sur une surface cylindrique interne d'une douille 435 de la partie mobile 423A.

La figure 6 montre une variante de la réalisation précédente où l'extrémité inférieure 203 du tube 201 comporte une collerette 450 épousant une partie de la surface conique de l'évasement en tronc de cône reliant l'alésage 421A à l'alésage 226 pour embout.

Cette collerette a pour but d'augmenter la solidarisation du tube 201 avec le manchon 405 en s'opposant à un déplacement longitudinal du tube dans le sens alésage 226-alésage 421A. Ici le tube 201 est simplement serti dans l'alésage 421A.

Les figures 7 et 8 montrent le conteneur 215 muni de son dispositif de maintien 220 de la figure 3 qui maintient la position du conteneur au niveau du col de l'utérus.

Le procédé de fécondation à l'aide de l'appareil de la figure 3 est décrit ci-après. L'homme de l'art pourra facilement transposer ce procédé à l'utilisation des autres appareils tels que déjà décrits en référence aux figures 4,5 ou 6.

Si le bouchon 204 est un bouchon tripartite du genre de celui que l'on trouve dans les paillettes françaises de CASSOU on insère un embout d'une seringue d'aspiration dans l'alésage 226 du manchon 205 et on aspire, par l'extrémité supérieure 202 du tube 201 formant conteneur 215, le milieu de culture, le ou les ovocyte(s) et les spermatozoïdes à l'intérieur du tube 201.

Si le bouchon n'est pas tripartite il est possible d'introduire à l'aide d'une seringue munie d'une aiguille le même contenu à l'intérieur du tube 201.

Le tube rempli est ensuite éventuellement bouché à son extrémité supérieure 202 à l'aide d'un bouchon en matériau biodégradable (non représenté aux figures 7 et 8).

L'appareil constitué par le centeneur 215 et son dispositif de maintien 220 est introduit dans le vagin du mammifère.

L'extrémité 202 du tube 201 est introduite dans l'orifice du col de l'utérus et le tout est poussé jusqu'à l'arrivée en butée de la molette 208 avec le col de l'utérus.

Le manchon 205 est alors maintenu immobile en rotation par une pince languette (non représentée) et la molette 208 est éloignée de l'épaulement 225 en la vissant sur le pas de vis 207, ce qui a pour effet d'augmenter la flèche de la courbure des deux languettes 222B qui appuient alors sur les parois internes du col de l'utérus fixant ainsi d'une manière amovible le dispositif de maintien 220 du conteneur 215 au col de l'utérus. La figure 7 montre que de préférence on s'arrange par le choix de la longueur du tube 201 pour que son extrémité supérieure 202 soit située à proximité du fond de l'utérus. Après avoir retiré la pince longuette le mammifère est laissé ainsi au repos pendant un délai de 15 à 55 heures pour obtenir un ou plusieurs embryons dans le tube 201.

La figure 7 montre cette phase de repos.

La figure 8 montre qu'après ce délai on éjecte le contenu du conteneur 215 dans le fond de la cavité utérine après fécondation du ou des ovocytes en repoussant le bouchon 204 vers l'extrémité supérieure 202 du tube 201 pour déverser le contenu du conteneur 215 directement en contact avec l'endomêtre en vue d'une nidation du ou des embryons. Ce repoussement du bouchon est effectué à l'aide d'un dispositif poussoir 500 comportant une tige souple 501 mobile dans une gaine 502. L'embout 503 de la gaine 502 vient s'adapter à frottement doux dans l'alésage 226.

La tige 501 a un diamètre inférieur à celui intérieur du tube 201 et une longueur adaptée par rapport à la longueur de la gaine 502, du bouchon 204 et du tube 201 pour que l'on ne puisse pas obtenir une sortie du bouchon 204 hors du tube 201 par l'extrémité 202. L'orifice rétréci 212 constitue également une sécurité pour le maintien du bouchon à l'intérieur du tube.

La molette 208 est ensuite dévissée de façon à détendre les languettes 222B et à libérer le dispositif de maintien 220. Tout l'appareillage est alors retiré des voies génitales du mammifère.

Les dispositifs de maintien ont été décrit en référence aux figures 3 à 6 avec deux languettes 222A. Il est possible de prévoir trois languettes ou plus régulièrement disposées autour du tube 201.

## Revendications

1. Procédé de fécondation intra-utérine de mammifère, caractérisé en ce qu'il comporte les étapes suivantes :
a) on prévoit un conteneur (15, 115, 215) apte à permettre son introduction et son logement dans la cavité utérine du mammifère ;
b) on remplit ce conteneur (15, 115, 215) avec un milieu de culture, au moins un ovocyte du mammifère et des spermatozoïdes ;
c) on loge ce conteneur (15, 115, 215) rempli dans la cavité utérine ;
d) on laisse le conteneur dans la cavité utérine pendant une durée déterminée pour obtenir la fécondation du ou des ovocytes dans le conteneur ;
e) on permet l'échappement du contenu du conteneur dans la cavité utérine.

2. Procédé suivant la revendication 1, caractérisé en ce qu'à l'étape e) l'échappement est fait par éjection du contenu du conteneur (215) dans le fond de la cavité utérine.

3. Procédé suivant la revendication 1, caractérisé en ce qu'à l'étape e) l'échappement du contenu du conteneur (15, 115) est effectué par suite de la biodégradation d'au moins une partie du conteneur (15, 115).

4. Procédé suivant la revendication 2, caractérisé en ce qu'on maintient la position du conteneur logé dans l'utérus, au niveau du col de l'utérus lors des étapes c) à e).

5. Procédé suivant la revendication 1 ou 3, caractérisé en ce qu'à l'étape c), on loge le conteneur (215) vers le fond de l'utérus.

6. Dispositif de fécondation de mammifère pour la mise en oeuvre du procédé selon l'une quelconque des revendications 1, 2 ou 4, dans lequel le conteneur comprend un tube (201) fermé à une de ses extrémités (203) dite inférieure, caractérisé en ce que ledit tube (201) est solidarisé vers son extrémité inférieure (203) à un dispositif de maintien (220, 320, 420) apte à être fixé d'une manière amovible au col de l'utérus du mammifère, après passage du tube par l'ouverture du col de l'utérus et logement du tube dans l'utérus.

7. Dispositif selon la revendication 6 caractérisé en ce que un bouchon (204) formant piston étanche ferme le tube à son extrémité inférieure.

8. Dispositif selon la revendication 6 ou 7, caractérisé en ce que le tube (201) a une collerette (450) à son extrémité inférieure (203).

9. Dispositif selon la revendication 6, 7 ou 8, caractérisé en ce que l'autre extrémité (202) du tube (201) dite supérieure définit une zone de sortie dirigée vers le fond de l'utérus lorsque le dispositif de maintien est fixé au col de l'utérus.

10. Dispositif selon la revendication 9, caractérisé en ce qu'il comporte en outre un moyen d'éjection du contenu du tube par la zone de sortie.

11. Dispositif selon la revendication 10, caractérisé en ce que l'extrémité supérieure (202) est fermée par un bouchon (301) en matière biodégradable apte à être expulsé par le déplacement du bouchon (204) formant piston.

12. Dispositif selon l'une quelconque des revendications 6 à 11, caractérisé en ce que le tube (201) est solidarisé au dispositif de maintien (220, 320, 420) à la faveur d'un alésage longitudinal traversant (221, 421) de celui-ci.

13. Dispositif selon l'une quelconque des revendications 6 à 12, caractérisé en ce que le dispositif de maintien (220, 320, 420) comporte une première partie (22) apte à être introduite dans le col de l'utérus et une seconde partie (223) apte à être disposée à l'extérieur du col et en butée contre celui-ci, la première partie comportant un élément extensible (222A) ayant une position de repos pour l'insertion dans le col et une deuxième position dans laquelle il s'appuie sur les parois internes du col de l'utérus.

14. Dispositif selon la revendication 13, caractérisé en ce que l'élément extensible (222A) est formé d'un matériau flexible à paroi mince apte à s'étendre radialement dans la deuxième position.

15. Dispositif selon la revendication 13 ou 14, caractérisé en ce que l'élément extensible (222A) comporte au moins deux languettes (222B) sensiblement longitudinales, chaque languette ayant une extrémité inférieure (222C) mobile et une extrémité supérieure (222D) fixe longitudinalement par rapport au tube (201).

16. Dispositif selon la revendication 15, caractérisé en ce que l'extrémité supérieure (222D) de l'élément extensible (222A) est fixée à une bague soudée (211) au tube (201).

17. Dispositif selon la revendication 15 ou 16, caractérisé en ce que le dispositif de maintien (220, 320, 420) comporte en outre une partie mobile (223A, 423A) pour contrôler la position longitudinale de l'extrémité inférieure (222C) de l'élément extensible (222A) et une partie fixe (222E, 422E) par rapport au tube (201) sur laquelle la partie mobile (223A, 423A) est adaptée à se déplacer.

18. Dispositif selon la revendication 17, caractérisé en ce que la partie fixe (222E, 422E) et la partie mobile (223A, 423A) comportent des pas de vis complémentaires (207, 207A, 431, 436) pour permettre à la partie mobile (223A, 423A) de tourner et de se déplacer longitudinalement par rapport à la partie fixe (222E, 422E).

19. Dispositif selon la revendication 18, caractérisé en ce que les pas de vis (431, 436) sont sur une surface externe de la partie fixe (422E) et sur une surface interne d'une douille (435) de la partie mobile (423A).

20. Dispositif selon la revendication 17 ou 18, caractérisé en ce que la partie mobile (223A) comporte une molette (208) formant écrou sur une extension longitudinale (224) de la partie fixe (222E) appartenant à ladite première partie (222) du dispositif de maintien (220, 320), extension (224) qui comporte sur une surface externe le pas de vis complémentaire (207) associé à celui (207A) de la molette (208).

21. Dispositif selon la revendication 20, caractérisé en ce que l'extrémité supérieure (222D) de l'élément extensible (222A) est attachée à l'extrémité supérieure de l'extension (224) de la partie fixe (222E).

22. Dispositif selon l'une quelconque des revendications 17 à 21, caractérisé en ce que la partie fixe (222E) comporte un alésage (226) de diamètre supérieur au diamètre du tube (201) pour recevoir un dispositif poussoir (500) adapté au déplacement du bouchon (204) vers l'extrémité supérieure (202) du tube (201).

23. Dispositif de fécondation de mammifère pour la mise en oeuvre du procédé selon l'une quelconque des revendications 1, 3 et 5 dans lequel le conteneur comprend un tube (11, 110), fermé à l'une au moins de ses extrémités, caractérisé en ce que le tube comporte une paroi (100) en un matériau biodégradable en un temps déterminé par son contenu prévu, à savoir ovocyte, spermatozoïdes et milieu de culture et par un environnement prévu intra-utérin, le tube ayant une longueur inférieure ou égale à la profondeur de la cavité utérine du mammifère.

24. Dispositif selon la revendication 23, caractérisé en ce que le tube est entièrement en un matériau biodégradable.

25. Dispositif selon la revendication 23 ou 24, caractérisé en ce que le matériau biodégradable est un polymère naturel du règne animal ou végétal.

26. Dispositif selon la revendication 25, caractérisé en ce que le polymère naturel est choisi parmi le collagène, le fibrinogène, et un sucre polymère.

27. Dispositif selon l'une quelconque des revendications 23 à 26, caractérisé en ce que la paroi (100) a une épaisseur telle que le temps déterminé de la biodégradation est compris entre 15 et 55 heures.

28. Dispositif selon l'une quelconque des revendications 23 à 27, caractérisé en ce que la paroi (100) a une épaisseur comprise entre 0,01 et 1 mm.

29. Dispositif selon l'une quelconque des revendications 23 à 28, caractérisé en ce qu'il comprend en outre un dispositif d'implantation (20) dudit tube (11) à l'intérieur de l'utérus du mammifère.

30. Dispositif selon la revendication 29, caractérisé en ce que le dispositif d'implantation (20) comprend un tuyau cylindrique (21) dont le diamètre intérieur est sensiblement égal au diamètre extérieur dudit tube (11).

## Claims

1. A process of intra-uterine fertilisation in mammals, characterised in that it comprises the following steps:
a) a container (15, 115, 215) is provided which is able to be inserted into and lodged in the uterine cavity of the mammal;
b) the container (15, 115, 215) is filled with a culture medium, at least one ovocyte of the mammal and spermatozoa;
c) the filled container (15, 115, 215) is lodged in the uterine cavity;
d) the container is left in the uterine cavity for a specific period of time in order to obtain fertilisation of the ovocyte(s) in the container;
e) the contents of the container are able to escape into the uterine cavity.

2. A process according to Claim 1, characterised in that in step (a) the escaping happens by the contents of the container (215) being ejected into the back of the uterine cavity.

3. A process according to Claim 1, characterised in that in step e) escape of the contents of the container (15, 115) takes place following biodegradation of at least a part of the container (15, 115).

4. A process according to Claim 2, characterised in that the container lodged in the uterus is kept in a position level with the neck of the uterus during the steps c) to e).

5. A process according to Claim 1 or Claim 3, characterised in that in step (c) the container (215) is lodged towards the back of the uterus.

6. A device for fertilisation in mammals for implementation of the process according to any one of Claims 1, 2 or 4, wherein the container comprises a tube (201) which is closed at one of its ends (203), called the bottom end, characterised in that said tube (201) is fixed towards its bottom end (203) to a holding device (220, 320, 420) which is capable of being immovably fixed to the neck of the mammal's uterus, after the tube has passed through the opening at the neck of the uterus and has been lodged in the uterus.

7. A device according to Claim 6, characterised in that a plug (204) which forms a seal-tight piston closes the lower end of the tube.

8. A device according to Claim 6 or Claim 7, characterised in that the tube (201) has a flange (450) at its bottom end (203).

9. A device according to Claim 6, 7 or 8, characterised in that the other end (202) of the tube (201), known as the upper end, defines an exit zone which is directed towards the bottom of the uterus when the holding device is fixed to the neck of the uterus.

10. A device according to Claim 9, characterised in that it furthermore comprises a means for ejecting the contents of the tube through the exit zone.

11. A device according to Claim 10, characterised in that the upper end (202) is closed by a plug (301) made of a biodegradable material and capable of being expelled by displacement of the piston-forming plug (204).

12. A device according to any one of Claims 6 to 11, characterised in that the tube (201) is fixed to the holding device (220, 320, 420) by virtue of a longitudinal bore (221, 421) passing through it.

13. A device according to any one of Claims 6 to 12, characterised in that the holding device (220, 320, 420) comprises a first portion (22) which is capable of being introduced into the neck of the uterus and a second portion (223) which is capable of being disposed outside the neck, abutting against it, the first portion comprising an expansible element (222A) with a rest position for insertion into the neck and a second position in which it bears against the inner walls of the neck of the uterus.

14. A device according to Claim 13, characterised in that the expansible element (222A) is formed of a flexible, thin-walled material which is capable of extending radially in the second position.

15. A device according to Claim 13 or Claim 14, characterized in that the expansible element (222A) comprises at least two substantially longitudinal tangs (222B), each tang having a lower end (222C) which is movable and an upper end (222D) which is fixed longitudinally relative to the tube (201).

16. A device according to Claim 15, characterized in that the upper end (222D) of the expansible element (222A) is fixed to a ring (211) which is soldered to the tube (201).

17. A device according to Claim 15 or 16, characterized in that the holding device (220, 320, 420) further comprises a movable portion (223A, 423A) to control the longitudinal position of the bottom end (222C) of the expansible element (222A) and a fixed portion (222E, 422E) relative to the tube (201) on which the movable portion (223A, 423A) is designed to be displaced.

18. A device according to Claim 17, characterized in that the fixed portion (222E, 422E) and the movable portion (223A, 423A) comprise complementary screwthreads (207, 207A, 431, 436) to enable the movable portion (223A, 423A) to rotate and be displaced longitudinally relative to the fixed portion (222E, 422E).

19. A device according to Claim 18, characterized in that the screwthreads (431, 436) are on an outer surface of the fixed portion (422E) and on an inner surface of a bush (435) of the movable portion (423A).

20. A device according to Claim 17 or 18, characterized in that the movable portion (223A) comprises a knurled disc (208) which forms a female screw on a longitudinal extension (224) of the fixed portion (222E) belonging to said first portion (222) of the holding device (220, 320), which extension (224) comprises on its outer surface the complementary screwthread (207) which is associated with that (207A) of the knurled disc (208).

21. A device according to Claim 20, characterized in that the upper end (222D) of the expansible element (222A) is attached to the upper end of the extension (224) of the fixed portion (222E).

22. A device according to any one of Claims 17 to 21, characterized in that the fixed portion (222E) comprises a bore (226) which is greater in diameter than the tube (201) to receive a pusher device (500) capable of displacing the plug (204) towards the upper end (202) of the tube (201).

23. A device for fertilisation in mammals, for implementation of the process according to any one of Claims 1, 3 and 5, wherein the container comprises a tube (11, 110) which is closed at at least one of its ends, characterized in that the tube comprises a wall (100) made of a material which is biodegradable over a period of time determined by the contents in the tube, namely ovocytes, spermatozoa and culture medium and by a given intra-uterine environment, the length of the tube being less than or equal to the depth of the mammal's uterine cavity.

24. A device according to Claim 23, characterized in that the tube is made entirely of a biodegradable material.

25. A device according to Claim 23 or Claim 24, characterised in that the biodegradable material is a natural polymer of the animal or vegetable kingdom.

26. A device according to Claim 25, characterized in that the natural polymer is selected from collagen, fibrinogen and a polymeric sugar.

27. A device according to any one of Claims 23 to 26, characterized in that the wall (100) is of a thickness such that the specific biodegradable time is between 15 and 55 hours.

28. A device according to any one of Claims 23 to 27, characterized in that the wall (100) is of a thickness of between 0.01 and 1 mm.

29. A device according to any one of Claims 23 to 28, characterized in that it further comprises a device (20) for implanting said tube (11) inside the uterus of the mammal.

30. A device according to Claim 29, characterised in that the implanting device (20) comprises a cylindrical tube (21), the internal diameter of which is substantially equal to the external diameter of said tube (11).

## Patentansprüche

1. Verfahren zur intra-uterinen Befruchtung, dadurch gekennzeichnet, daß es die folgenden Schritte aufweist:
a) man sieht einen Behälter (15, 115, 215) vor, der geeignet ist, seine Einführung und Aufnahme in dem Uterus zur ermöglichen:
b) man befüllt den Behälter (15, 115, 215) mit einem Nährboden, wenigstens einem Ovozyten und Samenzellen;
c) man verbringt den gefüllten Behälter (15, 115, 215) in den Uterus;
d) man beläßt den Behälter in dem Uterus während einer vorbestimmten Zeitdauer, um die Befruchtung des oder der Ovozyten in dem Behälter zu erzielen;
e) man ermöglicht den Austritt des Inhalts des Behälters in den Uterus.

2. Verfahren nach Anspruch 1, dadurch gekennzeichnet, daß bei dem Schritt e) der Austritt geschieht durch Ausstoß des Inhalts des Behälters (215) in dem Boden des Uterus.

3. Verfahren nach Anspruch 1, dadurch gekennzeichnet, daß bei Schritt e) der Austritt des Inhalts des Behälters (15, 115) geschieht in Folge des biologischen Abbaus wenigstens eines Teils des Behälters (15, 115).

4. Verfahren nach Anspruch 2, dadurch gekennzeichnet, daß man die Position des im Uterus aufgenommenen Behälters in Höhe des Gebärmutterhalses während der Schritte c) bis e) hält.

5. Verfahren nach Anspruch 1 oder 3, dadurch gekennzeichnet, daß man den Behälter (215) im Schritt c) zum Boden des Uterus hin aufnimmt.

6. Vorrichtung zur Befruchtung zur Durchführung des Verfahrens nach einem der Ansprüche 1, 2 oder 4, wobei der Behälter ein Rohr (201) aufweist, das an einem seiner Enden (203), unteres genannt, geschlossen ist, dadurch gekennzeichnet, daß das Rohr (201) zu seinem unteren Ende (203) hin mit einer Haltevorrichtung (220, 320, 420) verbunden ist, die geeignet ist, in **abnehmbarer** Weise mit dem Gebärmutterhals verbunden zu sein, und zwar nach Durchgang des Rohrs durch die Öffnung des Gebärmutterhalses und Aufnahme des Rohrs im Uterus.

7. Vorrichtung nach Anspruch 6, dadurch gekennzeichnet, daß ein einen engen Kolben bildender Stopfen (204) das Rohr an seinem unteren Ende Verschließt.

8. Vorrichtung nach Anspruch 6 oder 7, dadurch gekennzeichnet, daß das Rohr (201) an seinem unteren Ende (203) einen Flansch (450) hat.

9. Vorrichtung nach Anspruch 6, 7 oder 8, dadurch gekennzeichnet, daß das andere Ende (202) des Rohrs (201), oberes genannt, eine Austrittszone definiert, die zum Boden des Uterus gerichtet ist, wenn die Haltevorrichtung am Gebärmutterhals festgelegt ist.

10. Vorrichtung nach Anspruch 9, dadurch gekennzeichnet, daß sie ferner ein Mittel zum Ausstoß des Inhalts des Rohrs durch die Austrittszone aufweist.

11. Vorrichtung nach Anspruch 10, dadurch gekennzeichnet, daß das obere Ende (202) durch einen Stopfen (301) aus biologisch abbaubarem Material verschlossen ist, der geeignet ist, durch die Verschiebung des einen Kolben bildenden Stopfens (204) ausgestoßen zu werden.

12. Vorrichtung nach einem der Ansprüche 6 bis 11, dadurch gekennzeichnet, daß das Rohr (201) mit der Haltevorrichtung (220, 320, 420) durch eine Längsbohrung (221, 421) verbunden ist, die jene kreuzt.

13. Vorrichtung nach einem der Ansprüche 6 bis 12, dadurch gekennzeichnet, daß die Haltevorrichtung (220, 320, 420) einen ersten Abschnitt (22) aufweist, der geeignet ist, in den Gebärmutterhals eingeführt zu werden, und einen zweiten Abschnitt (223), der geeignet ist, außerhalb des Gebärmutterhalses und in Anlage gegen diesen angeordnet zu sein, wobei der erste Abschnitt ein erweiterbares Element (222A) aufweist, das eine Ruheposition hat zur Einführung in den Hals, und eine zweite Position, in der es sich gegen die Innenwände des Gebärmutterhalses abstützt.

14. Vorrichtung nach Anspruch 13, dadurch gekennzeichnet, daß das erweiterbare Element (222A) aus dünnwandigem biegsamem Material gebildet ist, das geeignet ist, sich radial in der zweiten Position zu weiten.

15. Vorrichtung nach Anspruch 13 oder 14, dadurch gekennzeichnet, daß das erweiterbare Element (222A) wenigstens zwei praktisch längsgerichtete Zungen (222B) aufweist, wobei jede Zunge ein unteres bewegliches Ende (222C) und ein oberes Ende (222D) hat, das in Längsrichtung bezüglich des Rohrs (201) fest ist.

16. Vorrichtung nach Anspruch 15, dadurch gekennzeichnet, daß das obere Ende (222D) des erweiterbaren Elementes (222A) an einem Ring befestigt ist, der mit dem Rohr (201) verschweißt ist.

17. Vorrichtung nach Anspruch 15 oder 16, dadurch gekennzeichnet, daß die Haltevorrichtung (220, 320, 420) ferner einen beweglichen Abschnitt (223A, 423A) aufweist, um die Längsposition des unteren Endes (222C) des erweiterbaren Elements (222A) zu steuern, und einen festen Abschnitt (222E, 422E) bezüglich des Rohrs (201), auf dem der bewegliche Abschnitt (223A, 423A) geeignet ist sich zu verschieben.

18. Vorrichtung nach Anspruch 17, dadurch gekennzeichnet, daß der feste Abschnitt (222E, 422E) und der bewegliche Abschnitt (223A, 423A) Gewindegänge von komplementären Schrauben (207, 207A, 431, 436) aufweisen, um zu ermöglichen, daß der bewegliche Abschnitt (223A, 423A) sich dreht und sich in Längsrichtung bezüglich des festen Abschnitts (222E, 422E) verschiebt.

19. Vorrichtung nach Anspruch 18, dadurch gekennzeichnet, daß die Schraubengänge (431, 436) auf einer Außenfläche des festen Abschnitts (422A) und auf einer Innenfläche einer Hülse (435) des beweglichen Abschnitts (423A) gelegen sind.

20. Vorrichtung nach Anspruch 17 oder 18, dadurch gekennzeichnet, daß der bewegliche Abschnitt (223A) eine Stellschraube (208) aufweist, die eine Mutter auf einer Längserweiterung (224) des festen Abschnitts (222E) bildet, zugehörig zu dem ersten Abschnitt (222) der Haltevorrichtung (220, 320), und zwar eine Erweiterung (224), die auf einer Außenfläche den komplementären Schraubengang (207) aufweist, der mit dem (207A) der Stellschraube (208) verbunden ist.

21. Vorrichtung nach Anspruch 20, dadurch gekennzeichnet, daß das obere Ende (222D) des erweiterbaren Elementes (222A) am oberen Ende der Erweiterung (224) des festen Abschnitts (222E) befestigt ist.

22. Vorrichtung nach einem der Ansprüche 17 bis 21, dadurch gekennzeichnet, daß der feste Abschnitt (222E) eine Bohrung (226) von größerem Durchmesser als der Durchmesser des Rohrs (201) aufweist, um eine Stößelvorrichtung (500) aufzunehmen, die geeignet ist zur Verschiebung des Stopfens (204) zum oberen Ende (202) des Rohrs (201).

23. Vorrichtung zur Befruchtung und zur Duchführung eines Verfahrens nach einem der Ansprüche 1, 3 und 5, wobei der Behälter ein Rohr (11, 110) aufweist, geschlossen wenigstens an einem seiner Enden, dadurch gekennzeichnet, daß das Rohr eine Wand aus biologisch abbaubarem Material aufweist zu einer Zeit, die durch seinen vorgesehenen Inhalt bestimmt ist, nämlich Ovozyten, Samenzellen und Nährboden und durch eine vorgesehene intrauterine Umgebung, wobei das Rohr eine Länge hat, die kleiner ist oder gleich der Tiefe des Uterus.

24. Vorrichtung nach Anspruch 23, dadurch gekennzeichnet, daß das Rohr ganz aus biologisch abbaubarem Material besteht.

25. Vorrichtung nach Anspruch 23 oder 24, dadurch gekennzeichnet, daß das biologisch abbaubare Material ein natürliches Polymer des Tier- oder Pflanzenreichs ist.

26. Vorrichtung nach Anspruch 25, dadurch gekennzeichnet, daß das natürliche Polymer gewählt ist unter Kollagen, Fibrinogen und einem polymeren Zucker.

27. Vorrichtung nach einem der Ansprüche 23 bis 26, dadurch gekennzeichnet, daß die Wand (100) eine derartige Dicke hat, daß die für den biologischen Abbau bestimmte Zeit zwischen 15 und 55 Stunden liegt.

28. Vorrichtung nach einem der Ansprüche 23 bis 27, dadurch gekennzeichnet, daß die Wand (100) einem Dicke zwischen 0,01 und 1 mm hat.

29. Vorrichtung nach einem der Ansprüche 23 bis 28, dadurch gekennzeichnet, daß sie ferner eine Vorrichtung zur Implantation (20) des Rohrs (11) im Inneren des Uterus aufweist.

30. Vorrichtung nach Anspruch 29, dadurch gekennzeichnet, daß die Implantationsvorrichtung (20) eine zylindrische Röhre (21) aufweist, deren Innendurchmesser praktisch gleich ist dem Außendurchmesser des Rohrs (11).
